# DEMANDE DE BREVET EUROPEEN

(11) **EP 1 810 701 A1**
(43) Date de publication de la demande: **25.07.2007**
(21) Numéro de dépôt: 07364002.1
(22) Date de dépôt: 17.01.2007
(51) Int. Cl.: A61L 27/04, A61L 27/30

(54) **Prothèses métalliques allégées, avec revêtements biocompatibles, pour la chirurgie orthopédique**

(30) Priorité: 18.01.2006 FR 0600432
(71) Demandeur: Dages, Didier, F-22360 Langueux (FR)
(72) Inventeur: Dages, Didier, F-22360 Langueux (FR)

(57) **Abrégé**

Procédés de réalisation de prothèses médicales pour la chirurgie de réparation osseuse permettant d'obtenir des implants mécaniquement résistants et très légers par l'emploi d'alliage d'aluminium pour la constitution du corps de prothèse (10, 20,21), ce corps étant ensuite revêtu sur l'ensemble de sa surface de matériau biocompatible. Le matériau biocompatible est soit un alliage chrome-cobalt (11, 12,22) dont l'épaisseur est réglable selon la fonctionnalité de chaque zone de la prothèse, soit une couche céramique (23) ; une prothèse peut, selon la fonctionnalité de ses zones, être recouverte dans certaines zones de l'alliage chrome-cobalt (22) et dans d'autres zones d'une couche céramique (23).

## Description

La présente invention concerne un procédé de fabrication de prothèses médicales et les produits obtenus par ce procédé.

La chirurgie orthopédique utilise, pour la réparation de traumatologie ou pour combattre les conséquences de fragilisation osseuse ou d'arthrose, des prothèses pour les articulations du genou, de la hanche, du coude ou de l'épaule ou d'autres articulations avec une tendance de forte croissance à l'utilisation de ces prothèses dans les pays dits « riches ».

Ces prothèses sont très souvent en métal, en concurrence avec d'autres matériaux (nacre, céramique) qui peuvent être plus favorables à la repousse de l'os mais sont d'un emploi plus délicat du fait de leur plus grande fragilité ; cette utilisation d'implants métalliques est donc en constant développement.

Parmi les matériaux métalliques biocompatibles, trois grandes familles d'alliages métalliques sont utilisées :
- les aciers inoxydables austénitiques au chrome - nickel - molybdène type « 316L » (alliage type Z4 CND 21.19), dont la densité est voisine de 8.
- le titane et ses alliages, dont la densité est voisine de 4,5 mais qui est difficile à mettre en oeuvre par moulage ou Forgeage compte tenu de sa température de fusion très élevée (> 1700°C) et de sa forte tendance à l'oxydation à chaud.
- les alliages chrome-cobalt, de composition pondérale environ 65% de cobalt, 28% de chrome et 6% de molybdène, possédant de très bonnes qualités tribologiques de résistance à l'abrasion et de faible frottement à l'état poli mais dont la densité est voisine de 8,3.

Ces deux dernières familles sont en constant développement ; les alliages chrome-cobalt sont plus faciles à mettre en oeuvre par moulage notamment du fait de leur température de fusion (environ 1300°C) moins élevée que le titane et ses alliages ; le titane et ses alliages ont l'avantage d'une densité 46% plus faible que les alliages chrome-cobalt.

Toutefois ces deux familles de matériaux ont une densité élevée en comparaison avec la matière osseuse qu'elle remplacent, ce qui constitue un handicap, notamment dans le cas où la pose de prothèse intervient en réparation orthopédique chez des sujets âgés moins aptes à la possibilité de repousse de l'os et dont la tonicité musculaire est moindre que des sujets plus jeunes réparés pour des causes traumatologiques (accidents de la route ou de la pratique sportive).

A titre d'exemple, une prothèse « condyle du genou » en alliage chrome-cobalt pèse couramment 300g et ce n'est pas la plus lourde.

Le but de la présente invention est de permettre l'utilisation d'alliages métalliques de densité beaucoup plus faible que les deux familles précédentes - essentiellement des alliages d'aluminium de densité voisine de 2,7 - peu coûteux et faciles à mettre en oeuvre par moulage ou forgeage et dont les caractéristiques mécaniques, bien que moindres que celles des 3 familles précédentes, sont largement suffisantes pour les applications orthopédiques.

Afin de conférer à ces matériaux la biocompatibilité et la résistance à l'abrasion au frottement et les qualités de glissement nécessaires à leur utilisation en tant qu'implants, la prothèse métallique - pleine ou évidée selon les possibilités de mise en oeuvre par moulage ou forgeage ou assemblage d'une combinaison de pièces obtenues séparément par ces deux techniques et selon la forme extérieure finale requise - ainsi réalisée en alliage d'aluminium est ensuite revêtue soit d'une couche d'épaisseur variable, selon les zones concernées de la prothèse, de 20 µm à 600µm d'alliage métallique chrome-cobalt au moyen d'un pistolet chalumeau de projection de poudre métallique soit d'une couche de céramique très dure déposée soit par projection de manière identique à l'industrie de la céramique (dépôt des couches d'émaillage de décoration) ou par trempage dans une solution de barbotine de manière identique à la création des moules céramique en fonderie à la cire perdue, Il est aussi possible de réaliser une combinaison de ces 2 types de revêtement, certaines zones étant revêtues de la couche minimum d'alliage chrome-cobalt assurant la biocompatibilité (zones « statiques » de la prothèse sans fonction de « glissement ») et les zones « fonctionnelles » de céramique.

Dans ce dernier cas, la cuisson de la couche céramique déposée ne se fait pas classiquement dans un four comme pour les industries céramique ou fonderie à la cire perdue, compte tenu de la relativement faible température de fusion des alliages d'aluminium (environ 620°C) mais selon un procédé spécial par rayonnement contrôlé décrit ci-après.

Quelques exemples de réalisations préférentielles mais non limitatives sont donnés ci-après, avec les 2 types possibles de revêtement pour la biocompatibilité et la résistance à l'abrasion au frottement ainsi qu'à la « corrosion» chimique du milieu dans lequel les prothèses sont implantées, ces 2 possibilités étant combinables sur une même prothèse selon les zones et les résultats recherchés.

Un condyle du genou, forme assez « compacte » difficile à évider, sera réalisé par moulage classique en alliage d'aluminium type AU4G par exemple et aura un poids de 95g environ au lieu des 300g de la même pièce en alliage chrome-cobalt.

Pour un revêtement métallique au chrome-cobalt, on utilise un chalumeau de projection thermique de poudres métalliques ; on utilisera de préférence une poudre assez fine de granulométrie 50 à 100µm, ce qui facilite le travail de dépôt en très fines couches, en opérant sur une seule « face » de la prothèse à chaque fois.

Chaque couche déposée a en effet une épaisseur faible (environ 20 à 80µm par couche) pour éviter un échauffement trop élevé de la pièce en alliage d'aluminium et on attend le refroidissement complet à la température ambiante de la prothèse avant dépôt d'une nouvelle couche ; ainsi qu'indiqué précédemment, les zones de la prothèse qui n'ont pas de fonctionnalité de « glissement » (par exemple celles qui correspondent à la partie de condyle fixée dans l'os du fémur) ne seront revêtues que du minimum d'épaisseur pour assurer la biocompatibilité. Les zones à fonctionnalité de « frottement glissement » seront revêtues, en plusieurs passages, d'une couche plus épaisse (jusqu'à 800µm) qui après polissage pour obtenir le «glacé » nécessaire à son bon fonctionnement aura une épaisseur finale d'environ 600µm permettant un long usage de la prothèse.

Le première couche déposée par projection entraîne une fusion localisée de la surface de la pièce en alliage d'aluminium sur une épaisseur d'environ 50µm ce qui assure une excellente adhérence de l'alliage biocompatible déposé ; l'excellente conductibilité thermique de l'alliage d'aluminium associé à la très faible épaisseur de dépôt par couche évite un échauffement trop élevé de la prothèse en alliage d'aluminium. En pratique, avec un peu d'expérience, l'échauffement de la prothèse ne dépasse pas 150°C environ.

Les couches successives déposées entraînant une fusion localisée sur une très faible épaisseur de la couche précédente assurent l'homogénéité et l'adhérence de l'ensemble de la couche de revêtement en alliage chrome-cobalt.

Le poids du revêtement en alliage chrome-cobalt sur la prothèse prête à la pose chirurgicale représente environ 5 à 10% maximum du poids de la pièce en alliage d'aluminium ; ce procédé permet ainsi de réaliser une prothèse ayant les mêmes propriétés fonctionnelles que celle en alliage chrome-cobalt massif pour une masse plus de trois fois inférieure.Cette masse est aussi plus de 36% plus faible que celle d'une coûteuse prothèse en alliage de titane.

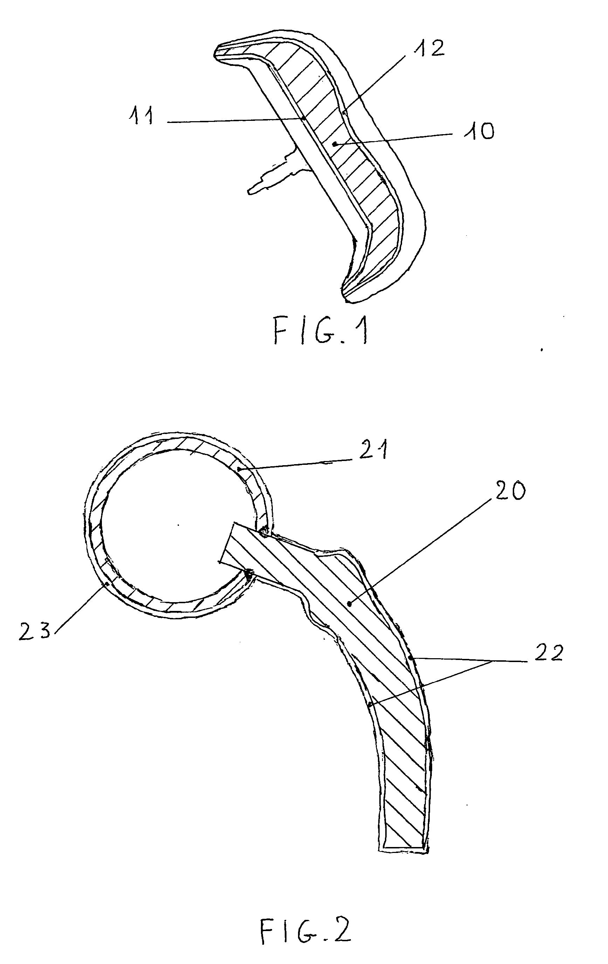

A l'usage, les différences de coefficient de dilatation entre l'alliage d'aluminium et le chrome-cobalt déposé ne sont pas gênantes car la température du corps humain est quasi constante. La figure 1 représente une coupe médiane selon l'axe du fémur sur laquelle elle est fixée de la prothèse de condyle du genou avec les différentes épaisseurs de couche de chrome-cobalt déposées selon les fonctionnalités des zones ; la partie hachurée large (10) correspond à la matière en alliage d'aluminium, la couche mince en chrome-cobalt d'environ 50µm en contact avec l'os du fémur est repérée (11) et la couche plus épaisse correspondant à la zone fonctionnelle de frottement glissement après polissage, d'une épaisseur environ 600µm, est repérée ( 12).

La représentation de la figure 1 n'est pas à l'échelle, afin de pouvoir symboliser les couches de chrome-cobalt de différentes épaisseurs.

Pour un revêtement céramique, la couche de céramique est soit déposée par pulvérisation avec un pistolet identique à ceux utilisés pour le dépôt « d'émaillage » sur des pièces en porcelaine ; le réservoir du pistolet à air comprimé est rempli d'un mélange de poudre de céramique - alumine essentiellement - et de liant silicate, mélange dilué dans l'eau.

La pièce en alliage d'aluminium est préalablement préférentiellement chauffée à environ 100°C pour accélérer le séchage de la couche céramique et son adhésion uniforme à la surface ; ce processus permet de déposer une couche céramique très fine (<50µm). On peut pulvériser plusieurs couches successivement si une épaisseur plus grande est localement nécessaire.

On peut aussi procéder au trempage de la pièce en alliage d'aluminium dans un bain de « barbotine » (mélange de poudre de céramique et liant silicate dilué dans l'eau) de viscosité correctement réglée, comme pour la réalisation des grappes de moules céramique dans le procédé de fonderie dit « à la cire perdue » ; dans ce cas toutefois la couche de céramique déposée avant cuisson est nettement plus épaisse (3 à 4 fois plus que par projection au pistolet) et sa compacité plus faible.

Dans les 2 cas, après séchage complet de la couche céramique déposée, il faut réaliser la cuisson de cette couche sans réchauffer exagérément la pièce en alliage d'aluminium qui supporte cette couche, c'est-à-dire sans dépasser 200°C environ dans la pièce en alliage d'aluminium. Ceci ne peut donc être réalisé dans un four du type de ceux utilisés pour la cuisson des couches céramique sur les pièces en porcelaine.

On utilise pour cela, avec un réglage expérimental, une exposition de très courte durée de chaque «face» de la prothèse revêtue céramique soit au rayonnement d'une source à haute température telle qu'une lampe à arc ou un résistor haute température en graphite ou carbure de silicium soit, avec beaucoup de « doigté », à la flamme non « dure » d'un chalumeau ou d'un brûleur à air induit. La durée d'exposition est expérimentalement limitée pour que la température moyenne de la pièce en alliage d'aluminium ne dépasse pas 150 à 200°C selon la forme de la prothèse et l'exposition durant cette courte durée est répétée plusieurs fois, après refroidissement entre chaque exposition, jusqu'à assurer la cuisson complète de la fine couche de céramique.

Les prothèses ainsi réalisées sont encore plus légères que les prothèses en alliage d'aluminium revêtues d'une couche métallique au chrome-cobalt puisque la densité de la couche céramique déposée est encore plus faible que celle de l'alliage d'aluminium.

Elles sont toutefois d'un emploi et d'une pose plus délicate que les prothèses revêtues chrome-cobalt compte tenu de la fragilité de la couche de céramique, matériau qui résiste médiocrement au choc.

On peut alors combiner les différentes méthodes exposées précédemment pour réaliser une prothèse où la partie revêtue céramique est une zone fonctionnelle non soumise au choc et peu « manipulée » lors de l'intervention chirurgicale de pose et les autres zones sont revêtues d'une couche en chrome-cobalt d'épaisseur adaptée selon leur fonctionnalité.

L'exemple suivant d'une « prothèse de hanche » (reconstitution d'une tête de fémur, côté articulation avec la hanche) illustre ces différentes possibilités : la figure 2 représente une coupe transversale « axiale » de cette prothèse.

La partie « massive » repérée 20 correspondant à la « tige » de la prothèse est obtenue par forgeage d'une ébauche en alliage AU4G ; la « tête » de prothèse repérée 21 correspondant à la forme hémisphérique qui se loge dans la hanche est évidée et obtenue.par moulage d'un alliage AU4G ; elle est ensuite assemblée à la tige 20 par soudage sous argon pour former la pièce complète résistante et la plus allégée possible.

La prothèse de hanche ainsi obtenue est ensuite revêtue d'une couche métallique chrome-cobalt (repérée 22) dans sa zone « tige » qui n'est pas soumise à un frottement de mouvement, couche d'une épaisseur la plus faible possible puisqu'elle n'a qu'un rôle de biocompatibilité ; la partie hémisphérique est ensuite revêtue d'une couche céramique (repérée 23). La représentation de la figure 2 n'est pas à l'échelle, afin de pouvoir symboliser les couches de chrome-cobalt et de céramique de différentes épaisseurs.

On obtient ainsi le meilleur ensemble au niveau résistance mécanique, légèreté, facilité de pose et qualité tribologique de la céramique pour la rotation de tête de fémur dans la hanche.

Cet ensemble de techniques permet de produire pour un coût plus faible que les prothèses métalliques « classique » des prothèses de toutes formes sans aucune limitation compte tenu des facilités de mise en forme des alliages d'aluminium (forgeage, moulage, assemblages mécano soudés) et dont le revêtement est ensuite adapté (céramique et/ou couche chrome-cobalt) selon l'usage et la fonctionnalité des zones de prothèse.

## Revendications

1. procédé de réalisation de prothèses médicales destinées à la chirurgie de réparation osseuse **caractérisé par** :
- la réalisation en alliage d'aluminium du corps de prothèse(10,20,21) en utilisant des techniques de forgeage et/ou de moulage ainsi que, si nécessaire selon la forme à réaliser et pour obtenir économiquement le meilleur ensemble possible en résistance mécanique pour le plus faible poids, assemblage mécano soudé(20,21).
- Le revêtement de tout ou partie de la surface du corps de prothèse ainsi réalisé par une couche d'alliage chrome-cobalt (11,12,22) d'épaisseur variable selon la fonctionnalité de la zone de prothèse concernée, revêtement conférant à la prothèse sa biocompatibilité avec le corps humain.
- Le revêtement de tout ou partie de la surface du corps de prothèse ainsi réalisé par une couche de céramique biocompatible (23).
Les deux types de revêtement (22,23) - alliage chrome-cobalt ou céramique- pouvant concerner une même prothèse dans différentes zones selon les fonctions de ces zones et assurant ensemble la couverture totale de la surface du corps de prothèse pour garantir la biocompatibilité de la prothèse prête à la pose chirurgicale.

2. procédé de revêtement d'une pièce en alliage d'aluminium par une couche d'alliage chrome-cobalt selon la revendication 1 **caractérisé en ce que** :
- l'épaisseur de chaque couche déposée est limitée grâce à l'emploi d'une poudre très fine associée au chalumeau de projection des petites gouttes d'alliage fondu chrome-cobalt sur la surface de la pièce en alliage d'aluminium.
- la couverture de la pièce à revêtir se fait en plusieurs étapes afin de limiter l'échauffement de la pièce en alliage d'aluminium à une température moyenne inférieure à 200°C après chaque passage de couche et avec attente d'un refroidissement complet à la température ambiante avant de recommencer l'opération sur une autre zone de surface de la pièce ou sur la même zone si une épaisseur de couche finale plus importante est fonctionnellement nécessaire.

3. procédé de revêtement d'une pièce en alliage d'aluminium par une couche de céramique selon la revendication 1 **caractérisé en ce que** :
- la couche de céramique déposée sur tout ou partie de la surface de la pièce en alliage d'aluminium soit par projection à l'aide d'un pistolet à air comprimé en un ou plusieurs passages ou par trempage dans une solution de « barbotine» » céramique est ensuite cuite par une succession de brèves expositions à une source rayonnante à haute température ou à la flamme non « dure » d'un chalumeau ou d'un brûleur à air induit,la durée de chaque exposition étant expérimentalement réglée pour que la température moyenne de la pièce en alliage d'aluminium ne dépasse pas 150°C environ et les expositions étant entrecoupées par des séquences de refroidissement de la pièce jusqu'à la température ambiante.

4. prothèse médicale pour la chirurgie de réparation osseuse réalisée selon l'une quelconque des revendications précédentes.
